# EUROPÄISCHE PATENTSCHRIFT

(11) **EP 0 691 887 B1**
(45) Veröffentlichungstag und Bekanntmachung des Hinweises auf die Patenterteilung: **04.06.1997**
(21) Anmeldenummer: 94913069.4
(22) Anmeldetag: 28.03.1994
(51) Int. Cl.: B01J 20/32, C12N 11/08

(54) **IMMOBILISIERTE ENZYME**
IMMOBILIZED ENZYMES
ENZYMES IMMOBILISEES

(30) Priorität: 03.04.1993 EP 93105560; 29.09.1993 DE 4333213
(43) Veröffentlichungstag der Anmeldung: 17.01.1996
(73) Patentinhaber: MERCK PATENT GmbH, 64293 Darmstadt (DE)
(72) Erfinder: MÜLLER, Egbert, D-64390 Erzhausen (DE); BADEL, Kerstin, D-41352 Korschenbroich (DE); MÜLLER, Andreas, D-65933 Frankfurt (DE); HERBERT, Stefan, D-36391 Sinntal 2 (DE); SEILER, Anja, D-63811 Stockstadt am Main (DE); HOLSCHUH, Karl, D-64342 Seeheim-Jugenheim (DE)
(86) Internationale Anmeldenummer: EP9400976
(87) Internationale Veröffentlichungsnummer: WO9422574

(56) Entgegenhaltungen:
- EP-A- 0 392 735
- EP-A- 0 392 783
- EP-A- 0 565 978
- WO-A-92/07879
- US-A- 4 332 694
- US-A- 4 737 560
- DATABASE WPI, Week 8121, Derwent Publications Ltd., London (GB); AN 81-37547d

## Beschreibung

Die Erfindung betrifft immobilisierte Enzyme und ihre Verwendung, sowie ihre Herstellung aus aktivierten Trägermaterialien.

Es ist bekannt, daß Enzyme auf Trägermaterialien mittels verschiedener Reaktanden, z.B. Oxiran-, Carbonylimidazol- oder Tresylderivaten, immobilisiert werden können. Immobilisierte Enzyme weisen oft eine verbesserte Stabilität auf. Sie lassen sich außerdem leicht aus dem Reaktionsansatz entfernen und können auch wiederverwendet werden. Eine weitere Verwendung von immobilisierten Enzymen ist die Bereitstellung von Enzymreaktoren, bei denen unter Durchflußbedingungen eine kontinuierliche Reaktionsführung möglich ist. Es hat sich jedoch gezeigt, daß Enzyme bei der Immobilisierung inaktiviert werden und die Ausbeute an gebundenem Enzym nach der Bindungsreaktion äußerst niedrig ist. Dabei sinkt insbesondere die Reaktivität gegen hochmolekulare Substrate (Molekulargewicht > 10³).

Aus der deutschen Patentanmeldung DE 26 21 974 ist ein Verfahren zur Immobilisierung von Enzymen unter Benutzung von Isocyanatverbindungen bekannt. Dieses Dokument lehrt, bevorzugterweise einen Schwingarm (spacer) zwischen Trägermaterial und Enzym einzufügen. Nach diesem Verfahren können verschiedene Enzyme immobilisiert werden, z.B. Proteinase K. Trotz des Schwingarmes ist die Aktivität dieser Präparationen gegen hochmolekulare Substrate im Vergleich zu den nativen Enzymen eingeschränkt. In WO 92/07 879 sind immobilisierte biologischaktive Materialien offenbart, die unter Verwendung von azlactonhaltigen Trägermaterialien, wie sie beispielsweise in EP 0 392 735 offenbart werden, zugänglich sind.

Als Beispiel für derartige Enzyme, die bevorzugt hochmolekulare Substrate umsetzen, und die mit bisher bekannten Methoden nicht immobilisiert werden können, sei die bakteriellle Nuklease hergestellt nach EP 0 229 866 (Nuklease S.m.), eine Nukleinsäurehydrolase, die RNA, sowie ein- und doppelsträngige DNA in kleine biologisch unwirksame Oligonukleotide hydrolysiert, genannt. Nuklease S.m. ist geeignet, in biologisch gewonnenen (pharmazeutischen) Wirkstoffen eventuell noch vorhandene Nukleinsäuren abzubauen und somit die unerwünschte Übertragung von genetischem Material auszuschließen. Ein derartiger Prozess wäre bevorzugt in einem Durchflußreaktor auszuführen. Voraussetzung dafür ist die Immobilisierung der Nuklease S.m. an einem geeigneten aktivierten Träger unter Erhalt der Reaktivität gegen hochmolekulare Nukleinsäuren.

Proteinase K wird aus dem Pilz Tritirachium album gewonnen und ist in DE 19 65 281 beschrieben. Wegen ihrer Fähigkeit, Proteine unspezifisch abzubauen, und wegen ihrer Stabilität wird Proteinase K bei der Isolierung und Aufreinigung von Nukleinsäuren benutzt, um den Proteinanteil zu entfernen. Dies schließt auch die Inaktivierung von Endo- und Exonukleasen ein. In der Molekularbiologie sind Umsetzungen an Nukleinsäuren mittels Enzymen üblich geworden. Zu derartigen Umsetzungen gehören beispielsweise die Erzeugung von Teilstücken mit definierten Endsequenzen mittels Restriktionsendonukleasen , die Vermehrung von Nukleinsäureabschnitten mittels der Polymerase-Ketten-Reaktion (PCR) unter Verwendung von Polymerasen und die Modifikation von Nukleinsäuren beispielsweise durch DNA-modifizierende Enzyme. Bei diesen Verfahren werden in den verschiedenen Verfahrensstufen Enzyme eingesetzt, die häufig jeweils vor der nächsten Verfahrensstufe inaktiviert werden müssen. Soweit für diese Inaktivierung Proteinasen benutzt werden, muß sichergestellt werden, daß die Proteinasen vor dem nächsten enzymatischen Verfahrensschritt inaktiviert oder entfernt werden, da sonst die nukleinsäuremodifizierenden Enzyme für den nächsten Reaktionsschrift vorzeitig inaktiviert würden. Bei der Verwendung von immobilisierten Proteinasen ist dies auf einfache Art möglich, wobei beispielsweise die aufwendige Extraktion der Nukleinsäuren unter Verwendung von organischen Lösungsmitteln vermieden wird. Voraussetzung ist aber, daß die Bindung der Proteinase an den Träger so fest ist, daß eine Kontamination der Reaktionslösung mit gelöster Proteinase unterbleibt. Anderenfalls würden die Enzyme, die für nachfolgende Reaktionsschritte benötigt werden, vorzeitig inaktiviert. Voraussetzung ist weiterhin, daß bei der Immobilisierung der Proteinase ihre Aktivität gegen hochmolekulare Substrate erhalten bleibt, da sonst die gewünschte Inaktivierung der Enzyme nicht möglich wäre.

Weitere Enzyme, bei deren Immobilisieruing ähnliche Probleme auftreten, sind dem Fachmann aus der Literatur bekannt.

In EP 0 172 579 werden Trägermaterialien beschrieben, die einen Kern aus Silikagel besitzen, auf den ein vernetztes Polymeres aufgebracht ist. Dieses Polymere kann als aktivierte Gruppe beispielsweise die Oxirangruppe enthalten. Die Bindung des Polymeren an den Träger erfolgt durch Reaktion eines Teiles der Oxirangruppen. Dabei entstehen Si-O-Bindungen, die empfindlich gegen Hydrolyse sind. Werden Enzyme an die verbleibenden Oxirangruppen gebunden, so ist der Zutritt von hochmolekularen Substraten wie Proteinen oder Nukleinsäuren durch die Vernetzung des Polymers behindert. Auch aus US 4,332,694 sind ähnliche oxiranhaltige Trägermaterialien bekannt

Es besteht also die Aufgabe, immobilisierte Enzyme bereitzustellen, bei denen die Auswaschrate vernachlässigbar ist und die eine uneingeschränkte Aktivität gegen hochmolekulare Substrate besitzen.

In der Anmeldung DE 43 10 964 sind oxiran- und azlactonhaltige aktivierte Trägermaterialien offenbart, bei denen Monomereinheiten der Formel I auf einen hydroxylgruppenhaltigen Basisträger aufgepfropft sind, 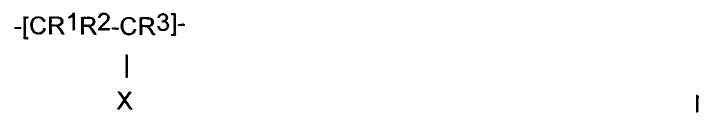 worin
- R¹, R² und R³: unabhängig voneinander
H oder CH₃
und
- X: eine aktivierte Gruppe enthaltend eine Azlacton- oder eine Oxirangruppe
bedeuten.

Dazu gehören insbesondere Verbindungen, in denen X einen Rest der Formel II bedeutet, 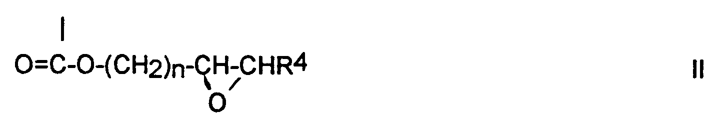 worin
- R⁴: H, C₁-C₅-Alkyl oder C₆-C₁₂-Aryl
und
- n: eine ganze Zahl zwischen 1 und 5
bedeuten.

Dazu gehören auch Verbindungen, in denen X einen Rest der Formel III bedeutet, 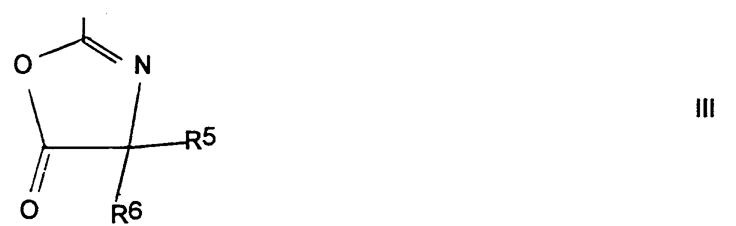 worin
- R⁵ und R⁶: unabhängig voneinander
H oder C₁-C₅-Alkyl
bedeuten.

Es wurde gefunden, daß sich diese aktivierten Trägermaterialien in an sich bekannter Weise benutzt werden können, um Enzyme zu immobilisieren. Diese erfindungsgemäßen Präparationen von immobilisierten Enzymen weisen verbesserte Eigenschaften auf.

Gegenstand der Erfindung sind somit immobilisierte Enzyme, erhältlich durch Reaktion von Enzymen mit aktivierten Trägermaterialien auf der Grundlage von hydroxylgruppenhaltigen Basisträgern, auf deren Oberflächen lineare Polymere über eine endständige Monomereinheit kovalent gebundenen sind, wobei die Polymeren Monomereinheiten der Formel I enthalten, 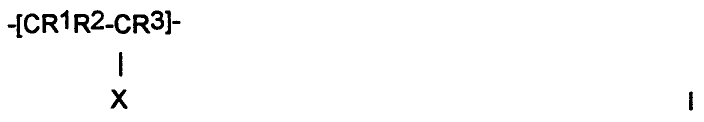 worin
- R¹, R² und R³: unabhängig voneinander
H oder CH₃
und
- X: eine aktivierte Gruppe enthaltend eine Azlacton- oder eine Oxirangruppe
bedeuten.

Gegenstand der Erfindung ist die Verwendung dieser aktivierten Trägermaterialien für die Immobilisierung von Enzymen.

Gegenstand der Erfindung sind auch Verfahren zur Immobilisierung von Enzymen, wobei Enzyme an die oben genannten aktivierten Trägermaterialien gebunden werden.

Gegenstand der Erfindung ist schließlich die Verwendung der erfindungsgemäßen immobilisierten Enzyme für enzymatische Umsetzungen.

Enzyme sind Proteine, die als Biokatalysatoren wirken. Es sind jedoch auch Nukleinsäurederivate bekannt, die katalytische Funktionen besitzen (sogenannte Ribozyme), und die erfindungsgemäß ebenfalls unter dem Begriff "Enzyme" subsummiert werden. Enzyme katalysieren spezifisch bestimmte Umsetzungen, z.B. Redox-Reaktionen oder Hydrolysen, bei denen bestimmte Substrate, beispielsweise Alkohole oder Nukleinsäuren, umgesetzt werden. Häufig werden von den Enzymen bei diesen Umsetzungen Cofaktoren benötigt. Im Stoffwechsel wirken häufig mehrere Enzyme zusammen, um Reaktionsketten zu katalysieren. Einzelheiten von enzymatisch katalysierten Reaktionen, den dabei beteiligten Enzymen und Cofaktoren, sowie von biochemischen Reaktionsketten sind dem Fachmann bekannt, Es ist weiterhin bekannt, daß Enzyme auf Trägermaterialien mittels verschiedener Reaktanden, z.B. Oxiran-, Carbonylimidazol- oder Tresylderivaten, immobilisiert werden können.

Wirken in einer Reaktionskette verschiedene Enzyme zusammen, so können diese auch in enger Nachbarschaft auf einem Träger immobilisiert werden, um für Zwischenprodukte die Diffusionswege kurz zu halten. Erfindungsgemäß können deswegen Enzyme einzeln immobilisiert werden, aber auch verschiedene, beispielsweise in Reaktionsketten kooperierende, Enzyme gemeinsam immobilisiert werden.

Dem Fachmann sind zahlreiche Enzyme bekannt, die Nukleinsäuren umsetzen, und die bei verschiedenen Verfahren, bei denen Nukleinsäuren verändert werden, eingesetzt werden können; dazu gehören beispielsweise: DNasen und RNasen, Nukleinsäurepolymerasen, Ligasen, DNA- und/oder RNA-umsetzende Phosphatasen, Kinasen und Transferasen sowie Restriktionsendonukleasen. Weiterhin gehören dazu nukleinsäure-modifizierende Enzyme, die beispielsweise Methylgruppen in eine Nukleinsäure einbauen.

Basisträger im Sinne der vorliegenden Erfindung sind Partikel, auf die Polymere aufgepfropft werden. Als Basisträger können allgemein übliche poröse oder unporöse Trägerteilchen eingesetzt werden, soweit sie primäre oder sekundäre aliphatische Hydroxylgruppen an ihrer Oberfläche aufweisen, oder in die nach an sich bekannten Verfahren aliphatische Hydroxylgruppen eingeführt werden können. Geeignet sind unter anderem Polysaccharide auf Agarose-Basis, Cellulose, Cellulosederivate und Polymere auf Dextran-Basis. Bevorzugt sind Polymere auf Polyvinylalkohol-Basis oder Copolymere aus (Meth)acrylatderivaten und Comonomeren mit aliphatischen Hydroxylgruppen. Insbesondere sind diolmodifizierte Kieselgele als Basisträger, sowie Mischpolymerisate auf Polyvinylbasis, die aliphatische Hydroxylgruppen enthalten, bevorzugt. Derartige Basisträger sind kommerziell erhältlich; z.B. Fractogel® TSK HW 65 (S) (Fa. E. Merck), ein poröses Mischpolymerisat auf Vinylbasis, das aliphatische Hydroxylgruppen enthält (1 mÄq OH/g), und LiChrospher® DIOL (Fa. E. Merck), ein diolsubstituiertes Kieselgel, ebenfalls mit aliphatischen Hydroxylgruppen.

Basisträger im weiteren Sinne sind aber auch membran- oder fadenförmige, sowie netz- oder gewebeähnliche oder schwammähnliche Materialien, die aliphatische Hydroxylgruppen enthalten, oder in die nach an sich bekannten Verfahren aliphatische Hydroxylgruppen eingeführt werden können. Diese Materialien können auch zusätzlich Stützelemente zur Verbesserung ihrer mechanischen Stabilität enthalten. Unter Verwendung ihrer aliphatischen Hydroxylgruppen können diese Materialien ebenfalls zu aktivierten Trägermaterialien umgesetzt werden. Dabei werden im wesentlichen dieselben Reaktionsfolgen angewandt, wie sie im folgenden für partikuläre Basisträger beschrieben werden.

Bei der Herstellung der in der vorliegenden Erfindung benutzten aktivierten Trägermaterialien wird die Polymerisation mit Cer(IV)-Ionen gestartet: G. Mino und S. Kaizerman (1958) J.Polymer Science 31, 242-243; G. Mino et al. (1959) J.Polymer Science 38, 393-401. Deren Reaktionsvariante wird in rein wäßriger, salpetersaurer Lösung ausgeführt und kann deswegen nur mit gut wasserlöslichen Monomeren ausgeführt werden. Es zeigte sich jedoch, daß die Umsetzung mit Cer(IV)- Salzen auch möglich ist, wenn als Lösungsmittel eine Mischung aus Wasser und organischen Lösungsmitteln, die keine Hydroxylgruppen enthalten, benutzt wird. Besonders bevorzugt sind dabei Dioxan oder Tetrahydrofuran. Der Anteil des organischen Lösungsmittels im Reaktionsansatz beträgt bevorzugt 10-80 Volumen-%, besonders bevorzugt 20-50 Volumen-%.

In Formel III sind die Reste R⁵ und R⁶ bevorzugt gleich, d.h. optischinaktive Verbindungen sind bevorzugt. Die Reste R⁵ und R⁶ bedeuten bevorzugt H, Methyl, Ethyl oder Propyl. Besonders bevorzugt bedeuten beide Reste entweder H oder Methyl; d.h. die Azlactonderivate, die sich vom Glycin und 2-Methylalanin ableiten, sind besonders bevorzugt.

Nach der Herstellung der immobilisierten Enzyme verbleiben im allgemeinen noch aktivierte Gruppierungen, die nicht reagiert haben. Üblicherweise werden diese Gruppen durch Umsetzung mit einem Überschuß einer wasserlöslichen Amino- oder Thiolverbindung umgesetzt. Bevorzugterweise wird für diesen Zweck Aminoethanol benutzt. Falls das immobiliserte Enzym hinreichend stabil ist, können die überschüssigen aktivierten Gruppierungen auch hydrolytisch mittels verdünnter sauerstoffhaltiger Mineralsäuren, wie z.B. Schwefel- oder Salpetersäure, entfernt werden. Bevorzugt ist dabei eine Umsetzung (Dauer: 1-5 Stunden) mit 0,5 M Schwefelsäure bei 35-60 °C.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen, Patente und Veröffentlichungen, sowie der korrespondierenden Anmeldungen EP 93105560.2, eingereicht am 03. April 1993, und DE 43 33 213, eingereicht am 29. September 1993, sind durch Bezugnahme in diese Anmeldung eingeführt.

Die folgenden Beispiele dienen der Erläuterung der Erfindung und stellen keine Begrenzung der Erfindung dar.

### Herstellungsbeispiele

In den folgenden Herstellungsbeispielen bedeutet Raumtemperatur (RT) 15-30 °C. Die Polymeriation wird in einem Dreihalskolben geeigneter Größe, der mit Rührer, Tropftrichter und Thermometer ausgerüstet ist, ausgeführt. Gewaschen wird durch Absaugen auf einer Nutsche.

### Beispiel 1: Herstellung eines oxiran-aktivierten Trägers ausgehend von Fractogel^{R}-TSK HW 65 (S)

Zu einer Suspension aus 50 ml sedimentiertem Fractogel^{R}-TSK HW 65 (S) und 25 ml Wasser werden mit 2 g Ammoniumcer(IV)nitrat (gelöst in 25 ml 2 M HNO₃) bei RT unter starkem Rühren vermischt. Nach 1 Minute erfolgt die Zugabe einer Lösung von 3 g (2,3-Epoxypropyl)-methacrylat in 30 ml Dioxan. Es wird 3 Stunden weitergerührt. Anschließend wird die Reaktionssuspension erst mit destilliertem Wasser und dann mit 0,05 M EDTA-Lösung gewaschen.

### Beispiel 2: Herstellung eines oxiran-aktivierten Trägers ausgehend von LiChrospher^{R}-Diol

Die Herstellung erfolgt entsprechend Beispiel 1, LiChrospher^{R}-DIOL (Partikelgröße 15-25 µm, Porengröße 80 nm) wird als Basisträger anstelle von Fractogel^{R}-TSK HW 65 (S) verwendet.

### Beispiel 3: Herstellung eines azlacton-aktivierten Trägers ausgehend von Fractogel^{R}-TSK HW 65 (S)

### Stufe 1: Pfropfpolymeres enthaltend Poly-(acryloylmethylalanin)

Für die Umsetzung wird das Ausgangsprodukt suspendiert und eine Monomer- und eine Initiatorlösung vorbereitet.
Monomerlösung: 5 g Acryloyl-2-methylalanin werden in 20 ml Wasser unter Zugabe von 4 M NaOH gelöst. Nach Zugabe von 50 ml Dioxan erwärmt man auf 45 °C. Der pH-Wert wird anschließend mit 25% HCL auf pH=1.5 eingestellt.
Initiatorlösung: 3.5 g Ammoniumcer(IV)nitrat werden in 50 ml 2 M HNO₃ gelöst.
Suspension des Ausgangsmaterials: 75 ml sedimentiertes Gel Fractogel^{R} TSK HW 65 (S) werden in 75 ml destilliertem Wasser suspendiert und die Suspension unter starkem Rühren (ca. 200 UpM) mit 100 ml Dioxan verdünnt.

Die Suspension des Ausgangsmaterials wird auf 45 °C erhitzt, die Apparatur evakuiert und mit Argon gespült.

Die Initiatorlösung wird zugegeben, 1 min gewartet und dann die auf 45 °C erwärmte Monomerlösung hinzugefügt. Der Ansatz wird 3 h bei 45 °C gerührt. Nach dem Abkühlen auf Raumtemperatur wird mit 0.2 M Sulfitlösung (pH=1), 0.5 M NaOH und Aceton gewaschen. Anschließend wird das Gel 24 h bei 60 °C getrocknet.

### Stufe 2: Zyklisierung zum Azlactonderivat

Das in Stufe 1 erhaltene Produkt wird mit 500 ml Essigsäureanhydrid versetzt und 2 Stunden bei 100 °C gerührt. Es wird abgesaugt und 24 Stunden bei 60 °C im Vakuumtrockenschrank getrocknet.

### Beispiel 4: Herstellung eines azlacton-aktivierten Trägers ausgehend von LiChrospher^{R}-DIOL

### Stufe 1: Pfropfpolymeres enthaltend Poly-(acryloylmethylalanin)

Für die Umsetzung wird das Ausgangsprodukt suspendiert und eine Monomer- und eine Initiatorlösung vorbereitet.
Monomerlösung: 5 g Acryloyl-2-methylalanin werden in 20 ml Wasser unter Zugabe von 4 M NaOH gelöst. Nach Zugabe von 50 ml Dioxan erwärmt man auf 45 °C. Der pH-Wert wird anschließend mit 25% HCL auf pH=1.5 eingestellt.
Initiatorlösung: 3.5 g Ammoniumcer(IV)nitrat werden in 50 ml 2 M HNO₃ gelöst.
Suspension des Ausgangsmaterials: 75 ml sedimentiertes Gel LiChrospher^{R}-DIOL werden in 75 ml destilliertem Wasser suspendiert und die Suspension unter starkem Rühren (ca. 200 UpM) mit 100 ml Dioxan verdünnt

Die Suspension des Ausgangsmaterials wird auf 45 °C erhitzt, die Apparatur evakuiert und mit Argon gespült.

Die Initiatorlösung wird zugegeben, 1 min gewartet und dann die auf 45 °C erwärmte Monomerlösung hinzugefügt. Der Ansatz wird 3 h bei 45 °C gerührt. Nach dem Abkühlen auf Raumtemperatur wird mit 0.2 M Sulfitlösung (pH=1) und mit Na-Phosphatpuffer (0.1 M; pH 7) gewaschen. Anschließend wird das Gel 24 h bei 60 °C getrocknet.

### Stufe 2: Zyklisierung zum Azlactonderivat

Das in Stufe 1 erhaltene Produkt wird mit 500 ml Essigsäureanhydrid versetzt und 2 Stunden bei 100 °C gerührt. Es wird abgesaugt und 24 Stunden bei 60 °C im Vakuumtrockenschrank getrocknet.

### Beispiel 5: Immobilisierung von Nuklease S.m. auf einem epoxy-aktivierten Träger

### Lösungen:

a) Starterlösung:
   3 g Ammoniumcer(IV)nitrat und 3 g Salpetersäure (Gehalt: 65%) werden in 190 ml Wasser gelöst.
b) Monomerlösung:
   6 g (2,3-Epoxypropyl)-methacrylat werden in 44 ml 1,4-Dioxan gelöst.
c) Puffer I:
   25 mM Tris/HCl, 0,5 mM MgCl₂, pH 8,0; 50 Vol.-% Ethylenglykol
d) Puffer L:
   25 mM Tris, 2,5 mM CaCl₂, pH 7,5; 50 Vol.-% Ethylenglykol

### Stufe 1: Pfropfen eines epoxidgruppenhaltigen Linearpolymers auf Fractogel^{R} TSK HW65(S)

100 ml sedimentiertes Fractogel^{R} TSK HW65(S) werden in 66 ml Wasser suspendiert. Die Starterlösung wird unter Rühren zugegeben und eine Minute gerührt. Anschließend wird die Monomerlösung unter Rühren zugegeben. Nach 1 h Rühren bei 180 U/min bei Raumtemperatur wird das Gel über eine Glasfilternutsche abgesaugt und mit jeweils 150 ml der nachfolgenden Waschlösungen behandelt: 4x Wasser, 3x Aceton, 3x Wasser, 1x Schwefelsäure 10%, 2x Wasser, 1x 0,2 M Phosphat-Puffer (pH 7), 3x Wasser, 3x Aceton, 1x Diethylether. Das aktivierte Gel wird bei 35 °C im Vakuumtrockenschrank getrocknet.

### Stufe 2: Reaktion der Nuklease S.m. mit dem aktivierten Gel

800 µl (1800 kU/ml) einer Lösung von Nuklease S.m. werden in 20 ml Puffer I gelöst. In dieser Lösung werden 2,5 g des aktivierten Gels aus Stufe 1 suspendiert. Das Reaktionsgemisch wird 20 h bei Raumtemperatur in einem Erlenmeyerkolben geschüttelt (200 UpM). Das Gel wird über eine Glasfilternutsche abgesaugt und mit je 20 ml der nachfolgend aufgeführten Waschlösungen behandelt: 2x mit Puffer I, 1x mit Wasser, 2x mit Puffer L. Das Gel wird abgesaugt und 3x mit 20 ml frischem sterilen Puffer L gewaschen und anschließend in Puffer L bei 4 °C gelagert.

### Beispiel 6: Immobilisierung von Proteinase K auf einem epoxy-aktivierten Träger

### Lösungen:

a) Starterlösung:
   15 g Ammoniumcer(IV)nitrat und 15 g Salpetersäure (Gehalt: 65%) werden in 950 ml Wasser gelöst.
b) Monomerlösung:
   30 g (2,3-Epoxypropyl)-methacrylat werden in 220 ml 1,4-Dioxan gelöst.
c) Puffer I:
   25 mM Barbiturat, 5 mM MgCl₂, pH 9,2
d) Puffer L:
   50 mM Tris, 5 mM CaCl₂, pH 7,5

### Stufe 1: Pfropfen eines epoxidgruppenhaltigen Linearpolymers auf Fractogel^{R} TSK HW65(S)

500 ml sedimentiertes Fractogel^{R} TSK HW65(S) werden in 330 ml Wasser suspendiert. Die Starterlösung wird unter Rühren zugegeben und eine Minute gerührt. Anschließend wird die Monomerlösung unter Rühren zugegeben. Nach 1 h Rühren bei 180 U/min bei Raumtemperatur wird das Gel über eine Glasfilternutsche abgesaugt und mit jeweils 500 ml der nachfolgenden Waschlösungen behandelt: 4x Wasser, 3x Aceton, 3x Wasser, 1x Schwefelsäure 10%, 2x Wasser, 2x 0,2 M Phosphat-Puffer (pH 7), 3x Wasser.

### Stufe 2: Reaktion der Proteinase K mit dem aktivierten Gel

25 g Proteinase K werden in 5000 ml Puffer I gelöst. In dieser Lösung wird das aktivierte Gel aus Stufe 1 suspendiert. Das Reaktionsgemisch wird 22 h bei Raumtemperatur in einem Dreihaiskolben gerührt. Nach 18 h wird der pH mit Natronlauge auf 9,2 nachgestellt. Das Gel wird über eine Glasfilternutsche abgesaugt und mit je 500 ml der nachfolgend aufgeführten Waschlösungen behandelt: 2x mit Puffer I, 1x mit 5 mM CaCl₂ in Wasser, 2x mit einer wäßrigen Lösung enthaltend 1 M NaCl und 5 im CaCl₂, 1x mit 5 mM CaCl₂ in Wasser, 2x mit einer wäßrigen Lösung enthaltend 0,1 M Natriumacetat (pH 4) und 5 mM CaCl₂, 1x mit 5 mM CaCl₂ in Wasser, 2x mit wäßriger 6 M Harnstofflösung, 3x mit 5 mM CaCl₂ in Wasser, 2x mit Puffer L. Das Gel wird abgesaugt und 3x mit 500 ml frischem sterilen Puffer L gewaschen und anschließend in Puffer L bei 4 °C gelagert.

Es ergibt sich eine Beladung von 2,9 mg Proteinase K/g Gel (Trockengewicht). Die Aktiviät beträgt 1,5 U/mg Gel (Trockengewicht; Acetyltyrosinethylester (ATEE) als Substrat), beziehungsweise 0,445 U/mg Gel (Trockengewicht; Resorufin-Casein als Substrat).

Aus den obigen Beispielen ist ersichtlich, daß die erfindungsgemäßen aktivierten Trägermaterialien hervorragend zur Immobilisierung von Enzymen geeignet sind. Wie die folgenden Anwendungsbeispiele zeigen, bleibt die enzymatische Aktivität, insbesondere gegen hochmolekulare Substrate, weitgehend erhalten.

### Anwendungsbeispiele

### Anwendungsbeispiel A: Bestimmung der Aktivität von immobilisierter Nuklease S.m.

DNA-Präparationen mit verschiedenem Molekulargewicht (Hind: Hind III Fragment von λ-DNA, pBR: Plasmid pBR 322, λ-DNA: DNA des Phagen λ) werden mit immobilisierter Nuklease S.m., hergestellt nach Beispiel 5, inkubiert (Schütteln bei 37 °C) und zu verschiedenen Zeiten (1, 4, 16 min.) der Anteil von hochmolekularen Substrat im Reaktionsüberstand durch Elektrophorese auf Agarosegel (0,8%) bestimmt. Die immobilisierte Nuklease S.m. ist in Tris- Puffer pH 8,0 (50 mM Tris, 1 mM MgCl₂, 50 Vol.-% Glycerin, 0,5 Vol.-% Mercaptoethanol) suspendiert; es werden jeweils 10 µl Suspension eingesetzt. Diese Suspension wird mit 50 µl der jeweiligen DNA-Lösung versetzt und inkubiert.

| Zeit: | 1 | 4 | 16 Stunden |
|---|---|---|---|
| Substrat: | | | |
| Hind¹⁾ | -- | -- | -- |
| pBR²⁾ | -- | -- | -- |
| λ-DNA³⁾ | ++ | + | - |
| Erläuterung: ++ hochmolekulare Anteile im wesentlichen vorhanden + hochmolekulare Anteile deutlich abgebaut - hochmolekulare Anteile im wesentlichen abgebaut -- keine hochmolekularen Anteile mehr nachweisbar | | | |

| | | | |
|---|---|---|---|
| 1) Fragmente mit 23130, 9416, 6557, 4361, 2322, 2027, 564 und 125 Basenpaaren Länge | | | |
| 2) 4361 Basenpaare | | | |
| 3) 48502 Basenpaare | | | |

### Anwendungsbeispiel B: Inaktivierung verschiedener Restriktionsendonukleasen

0,01 ml suspendierte immobilisierte Proteinase K, hergestellt nach Beispiel 6 werden in ein 1,5 ml Mikrozentrifugenröhrchen pipttiert. 0,2 ml Waschpuffer (50 mM Tris-HCl, pH 7,5) werden zugefügt, die Suspension wird kräftig gemischt (Vortex-Mischer) und anschließend 30 Sekunden zentrifugiert (Tischzentrifuge). Der Überstand wird abpipettiert und verworfen. Anschließend werden 0,05 ml der zu inaktivierenden Enzymlösung (z.B. Restriktionsendonuklease; jeweils 5, 10 und 20 U) zugefügt und die Suspension leicht geschüttelt (Vortex-Mischer). Nach zwei Minuten bei Raumtemperatur wird die immobilisierte Proteinase K abzentrifugiert (30 Sekunden, Tischzentrifuge). Es wird untersucht, ob der Überstand noch nachweisbare Aktivitäten des zu inaktivierenden Enzymes aufweist. Dazu werden 0,04 ml des Überstandes mit 0,01 ml λ-DNA (0,001 mg) zwei Stunden bei 37 °C inkubiert. Anschließend wird durch Elektrophorese auf 0,8 % Agarose-Gel untersucht, ob die DNA intakt geblieben ist.

Folgende Aktivitäten (U) der verschiedenen Enzyme werden mindestens inaktiviert:

| | | | | | | | | |
|---|---|---|---|---|---|---|---|---|
| Enzym: | AccI | AspI | BamHI | BanHI | BglI | Cla | EcoRI | HincII |
| Menge: | 5 | 20 | 5 | 20 | 20 | 20 | 20 | 20 |
| Enzym: | HindIII | KpnI | PstI | SalI | SmaI | SphI | TaqI | XbaI |
| Menge: | 5 | 20 | 10 | 20 | 5 | 10 | 5 | 5 |

### Anwendungsbeispiel C: Wiederverwendbarkeit der trägergebundenen Proteinase K

Unter Verwendung von 10 U PstI wird der Versuch, wie in Anwendungsbeispiel B beschrieben, wiederholt. Nach dem Abtrennen des Überstandes wird die immobilisierte Proteinase K jedoch insgesamt dreißigmal wiederverwendet. Die Überstände nach 1, 5, 10, 15, 20, 25 und 30 solchen Inaktivierungsschritten werden analysiert. In allen Fällen blieb die Proben- DNA intakt; d.h. die Restriktionsendonuklease wurde vollständig inaktiviert.

### Anwendungsbeispiel D: Inaktivierung von RNase

0,0 1 ml suspendierte immobilisierte Proteinase K, hergestellt nach Beispiel 6 werden in ein 1,5 ml Mikrozentrifugenröhrchen pipettiert. 0,2 ml Waschpuffer (50 mM Tris-HCl; pH 7,5) werden zugefügt, die Suspension wird kräftig gemischt (Vortex-Mischer) und anschließend 30 Sekunden zentrifugiert (Tischzentrifuge). Der Überstand wird abpipettiert und verworfen. Anschließend werden je 0,05 ml der zu inaktivierenden Enzymlösung (RNase A; jeweils 0,05; 0,1 und 0,2 U) zugefügt und die Suspension leicht geschüttelt (Vortex-Mischer). Nach zwei Minuten bei Raumtemperatur wird die immobilisierte Proteinase K abzentrifugiert (30 Sekunden, Tischzentrifuge). Es wird untersucht, ob der Überstand noch nachweisbare RNas-Aktivität aufweist. Dazu werden 0,04 ml des Überstandes mit 0,01 ml RNA aus Hefe (0,005 mg) eine Stunde bei 37 °C inkubiert. Anschliessend wird durch Elektrophorese untersucht, ob die RNA intakt geblieben ist.

### Anwendungsbeispiel E: Aktivität gegen Resorufin-Casein

Nach Standardverfahren unter Benutzung von Resorufin-Casein als (makromolekulares) Substrat wird die spezifische Aktivität von immobilisierter Proteinase K, hergestellt nach Beispiel 6, mit der von nativer Proteinase K verglichen, wobei die Beladung des Trägers mit Proteinase K berücksichtigt wird:

| | spezifische Aktivität (U/mg) |
|---|---|
| immobilisiert: | 153 |
| nativ: | 154 |

### Anwendungsbeispiel F: Ausblutverhalten

Immobilisierte Proteinase K, hergestellt nach Beispiel 6 und nach dem Stand der Technik werden nach einem Standardverfahren bei pH 7 drei Stunden gerührt; anschließend wird die Aktivität der Proteinase K im Überstand unter Verwendung von ATEE als Substrat gemessen. Die Ergebnisse (bezogen auf das Gelgewicht, beziehungsweise -volumen) werden mit der im Gel gebundenen Aktivität an Proteinase K verglichen:

| Material: | Stand der Technik | Beispiel 6 |
|---|---|---|
| Aktivität | U/ml | U/mg |
| Gel | 7,9 | 1,5 |
| ausgewaschen | 0,38 | 0,005 |
| % | 4,81 | 0,33 |

Aus den obigen Beispielen ist ersichtlich, daß bei der erfindungsgemäßen Immobilisierung beispielsweise von Proteinase K die Aktivität gegen hochmolekulare Substrate weitgehend erhalten bleibt, und daß im Vergelich zum Stand der Technik nur vernachlässigbare Mengen an Enzym ausgewaschen werden. Dabei ist zu berücksichtigen, daß in Anwendungsbeispiel F die Reaktionszeit das Sechzigfache der zur Inaktivierung der Endonukleasen üblichen Reaktionszeit betrug.

## Patentansprüche

1. Immobilisiertes Enzym, erhältlich durch Reaktion eines Enzymes mit aktivierten Trägermaterialien auf der Grundlage von hydroxylgruppenhaltigen Basisträgern, dadurch gekennzeichnet, daß auf der Oberfläche des Basisträgers lineare Polymere über eine endständige Monomereinheit kovalent gebunden sind, und daß die Polymeren Monomereinheiten der Formel I enthalten, 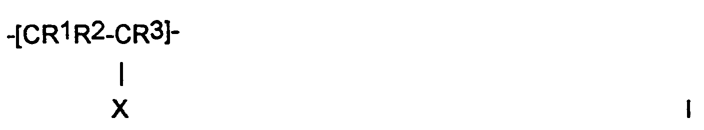 worin
R¹, R² und R³ unabhängig voneinander
H oder CH₃
und
X eine aktivierte Gruppe enthaltend eine Azlacton- oder eine Oxirangruppe
bedeuten.

2. Immobilisiertes Enzym nach Anspruch 1, dadurch gekennzeichnet, daß X in Formel I einen Rest der Formel II bedeutet, 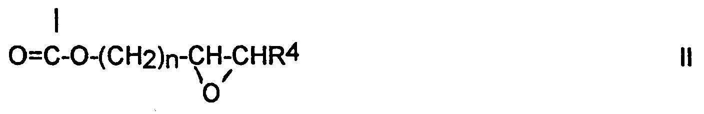 worin
R⁴ H, C₁-C₅-Alkyl oder C₆-C₁₂-Aryl
und
n eine ganze Zahl zwischen 1 und 5
bedeuten.

3. Immobilisiertes Enzym nach Anspruch 1, dadurch gekennzeichnet, daß X in Formel I einen Rest nach Formel III bedeutet, 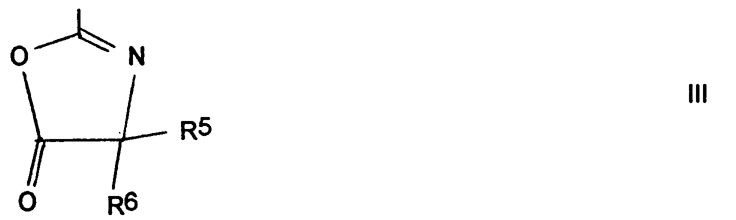 worin
R⁵ und R⁶ unabhängig voneinander
H oder C₁-C₅-Alkyl
bedeuten.

4. Verwendung eines aktivierten Trägermaterials auf der Grundlage von hydroxylgruppenhaltigen Basisträgern, auf deren Oberfläche lineare Polymere über eine endständige Monomereinheit kovalent gebunden sind, wobei die Polymeren Monomereinheiten der Formel I enthalten, 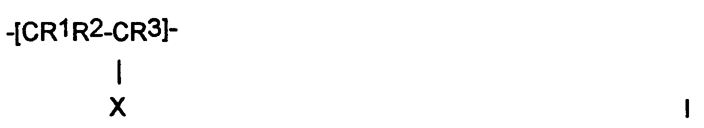 worin
R¹, R² und R³ unabhängig voneinander
H oder CH₃
und
X eine aktivierte Gruppe enthaltend eine Azlacton- oder eine Oxirangruppe
bedeuten, für die Immobilisierung von Enzymen.

5. Verfahren zur Immobilisierung von Enzymen dadurch gekennzeichnet, daß ein Enzym an aktivierte Trägermaterialien auf der Grundlage von hydroxylgruppenhaltigen Basisträgern, auf deren Oberfläche lineare Polymere über eine endständige Monomereinheit kovalent gebunden sind, wobei die Polymeren Monomereinheiten der Formel I enthalten, 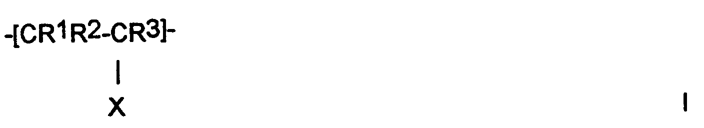 worin
R¹, R² und R³ unabhängig voneinander
H oder CH₃
und
X eine aktivierte Gruppe enthaltend eine Azlacton- oder eine Oxirangruppe
bedeuten, gebunden wird.

6. Verfahren nach Anspruch 5, dadurch gekennzeichnet, daß X in Formel I einen Rest der Formel II bedeutet, 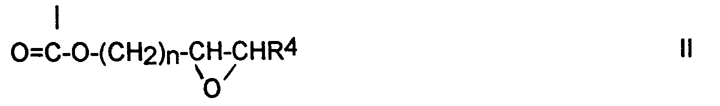 worin
R⁴ H, C₁-C₅-Alkyl oder C₆-C₁₂-Aryl
und
n eine ganze Zahl zwischen 1 und 5
bedeuten.

7. Immobilisierte Nuklease S.m., herstellbar nach einem Verfahren nach Anspruch 5 oder 6.

8. Verwendung von immobilisierter Nuklease S.m. nach Anspruch 7 zur Hydrolyse von Nukleinsäuren.

9. Immobilisierte Proteinase K, herstellbar nach einem Verfahren nach Anspruch 5 oder 6.

10. Verwendung von immobilisierter Proteinase K nach Anspruch 9 bei der Aufreinigung von Nukleinsäuren.

11. Verwendung nach Anspruch 10 zur Inaktivierung von Nukleasen, insbesondere Restriktionsendonukleasen, und von DNA-modifizierenden Enzymen.

## Claims

1. Immobilized enzyme which can be obtained by reacting an enzyme with activated support materials which are based on hydroxyl group-containing basic supports, characterized in that linear polymers are covalently bound, via a terminal monomer unit, onto the surface of the basic support, and in that the polymers contain monomeric units of the formula I, 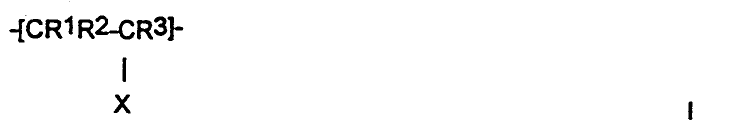 in which
R¹, R² and R³ are, independently of each other
H or CH₃, and
X is an activated group which contains an azlactone or an oxirane group.

2. Immobilized enzyme according to Claim 1, characterized in that X in formula I is a radical of the formula II 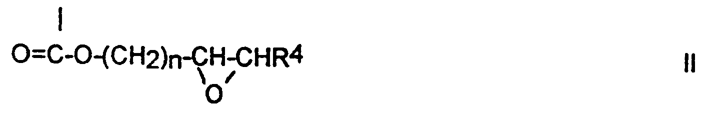 in which
R⁴ is H, C₁₋C₅-alkyl or C₆-C₁₂-aryl,
and
n is an integer between 1 and 5.

3. Immobilized enzyme according to Claim 1, characterized in that X in formula I is a radical according to formula III 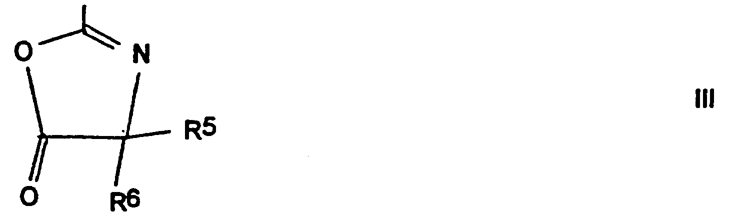 in which
R⁵ and R⁶ are, independently of each other,
H or C₁-C₅-alkyl.

4. Use of an activated support material which is based on hydroxyl group-containing basic supports, on whose surface linear polymers are covalently bound via a terminal monomer unit, with the polymers containing monomer units of the formula I 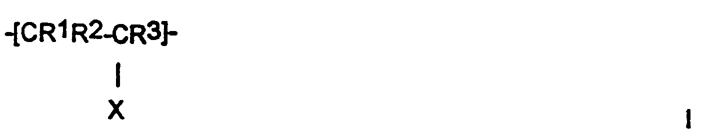 in which
R¹, R² and R³ are, independently of each other,
H or CH₃, and
X is an activated group which contains an azlactone group or an oxirane group,
for immobilizing enzymes.

5. Method for immobilizing enzymes, characterized in that an enzyme is bound to activated support materials which are based on hydroxyl group-containing basic supports, on whose surface linear polymers are covalently bound via a terminal monomer unit, with the polymers containing monomer units of the formula I 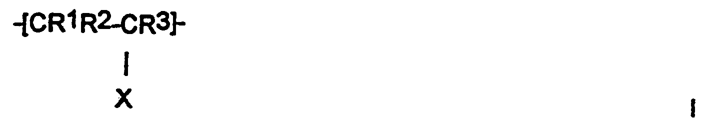 in which
R¹, R² and R³ are, independently of each other,
H or CH₃,
and
X is an activated group which contains an azlactone group or an oxirane group.

6. Method according to Claim 5, characterized in that X in formula I is a radical of the formula II 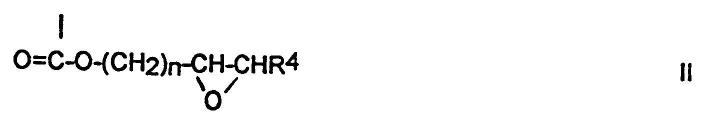 in which
R⁴ is H, C₁-C₅-alkyl or C₆-C₁₂-aryl,
and
n is an integer between 1 and 5.

7. Immobilized S.m. nuclease which can be prepared by a method according to Claim 5 or 6.

8. Use of immobilized S.m. nuclease according to Claim 7 for hydrolysing nucleic acids.

9. Immobilized proteinase K, which can be prepared by a method according to Claim 5 or 6.

10. Use of immobilized proteinase K according to Claim 9 in the purification of nucleic acids.

11. Use according to Claim 10 for inactivating nucleases, in particular restriction endonucleases, and for inactivating DNA-modifying enzymes.

## Revendications

1. Enzyme immobilisée, pouvant être obtenue par la réaction d'une enzyme avec des substances de support activées à base de supports de base contenant des groupes hydroxy, caractérisée en ce que, à la surface du support de base, des polymères linéaires sont liés par covalence, par l'intermédiaire d'un motif monomère en bout de chaîne, et en ce que les polymères contiennent des motifs monomères de formule I, 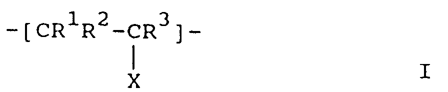 dans laquelle
R¹, R² et R³ représentent, indépendamment les uns des autres, H ou CH₃, et
X représente un groupe activé contenant un groupe azlactone ou oxiranne.

2. Enzyme immobilisée selon la revendication 1, caractérisée en ce que X dans la formule I représente un radical de formule II, 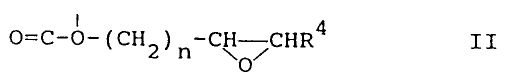 dans laquelle
R⁴ représente H ou un groupe alkyle en C₁-C₅ ou aryle en C₆-C₁₂, et
n est un nombre entier compris entre 1 et 5.

3. Enzyme immobilisée selon la revendication 1, caractérisée en ce que X dans la formule I représente un radical de formule III 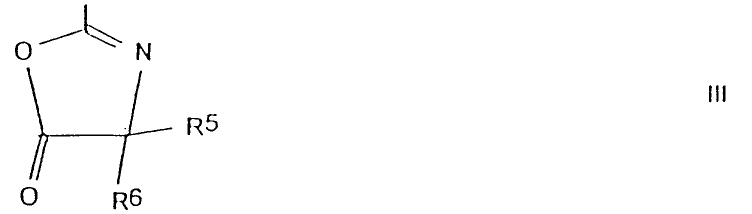 dans laquelle
R⁵ et R⁶ représentent, indépendamment l'un de l'autre, H ou groupe alkyle en C₁-C₅.

4. Utilisation d'une substance de support activée à base de supports de base contenant des groupes hydroxy, à la surface desquels des polymères linéaires sont liés par covalence, par l'intermédiaire d'un motif monomère en bout de chaîne, les polymères contenant des motifs monomères de formule I, 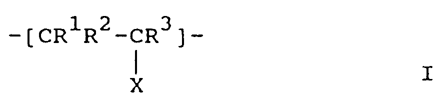 dans laquelle
R¹, R² et R³ représentent, indépendamment les uns des autres, H ou CH₃, et
X représente un groupe activé contenant un groupe azlactone ou oxiranne,
pour l'immobilisation d'enzymes.

5. Procédé pour l'immobilisation d'enzymes, caractérisé en ce qu'une enzyme est fixée à des substances de support activées à base de supports de base contenant des groupes hydroxy, à la surface desquels des polymères linéaires sont liés par covalence, par l'intermédiaire d'un motif monomère en bout de chaîne, les polymères contenant des motifs monomères de formule I, 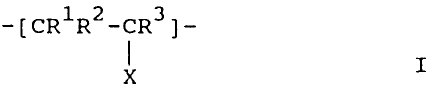 dans laquelle
R¹, R² et R³ représentent, indépendamment les uns des autres, H ou CH₃, et
X représente un groupe activé contenant un groupe azlactone ou oxiranne.

6. Procédé selon la revendication 5, caractérisé en ce que X dans la formule I représente un radical de formule II, 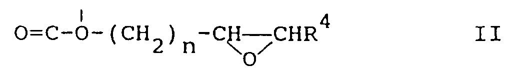 dans laquelle
R⁴ représente H ou un groupe alkyle en C₁-C₅ ou aryle en C₆-C₁₂, et
n est un nombre entier compris entre 1 et 5.

7. Nucléase S.m. immobilisée, pouvant être préparée selon un procédé conforme à la revendication 5 ou 6.

8. Utilisation de la nucléase S.m. immobilisée selon la revendication 7, pour l'hydrolyse d'acides nucléiques.

9. Protéinase K immobilisée, pouvant être préparée selon un procédé conforme à la revendication 5 ou 6.

10. Utilisation de la protéinase K immobilisée selon la revendication 9, pour la purification d'acides nucléiques.

11. Utilisation selon la revendication 10, pour l'inactivation de nucléases, en particulier d'endonucléases de restriction, et d'enzymes modifiant l'ADN.
